# EUROPEAN PATENT SPECIFICATION

(11) **EP 2 347 003 B1**
(45) Date of publication and mention of the grant of the patent: **10.06.2020**
(21) Application number: 09824038.5
(22) Date of filing: 23.10.2009
(51) Int. Cl.: C12P 19/18, C12N 9/24, C07G 3/00, C07H 1/00

(54) **GALACTOSE ALPHA (1-3) GALACTOSE COMPOSITIONS**
GALACTOSE-ALPHA (1-3)-GALACTOSE-ZUSAMMENSETZUNGEN
COMPOSITIONS DE GALACTOSE-ALPHA (1-3)-GALACTOSE

(30) Priority: 29.10.2008 US 109296 P
(43) Date of publication of application: 27.07.2011
(73) Proprietor: Janssen Biotech, Inc., Horsham, PA 19044 (US)
(72) Inventor: RAJU, T., Shantha, Radnor, PA 19087 (US); SCALLON, Bernard, J., Randor, PA 19087 (US)
(74) Representative: Goodfellow, Hugh Robin
(86) International application number: PCT/US2009/061810
(87) International publication number: WO 2010/051227

(56) References cited:
- WO-A1-2009/146362
- US-A- 5 922 577
- US-A- 5 922 577
- US-A1- 2004 191 256
- US-B1- 6 406 894
- MOGEMARK M ET AL: "Solid-phase synthesis of [alpha]-Gal epitopes: On-resin analysis of solid-phase oligosaccharide synthesis with <19>F NMR spectroscopy", JOURNAL OF ORGANIC CHEMISTRY 20030919 AMERICAN CHEMICAL SOCIETY US, vol. 68, no. 19, 19 September 2003 (2003-09-19), pages 7281-7288, XP002750711,

## Description

### Field of the Invention

The invention is directed to a method of enzymatic synthesis of oligosaccharide structures. In particular, the invention provides a method for synthesizing antibody compositions, comprising a terminal Gal-alpha(1,3)-Gal-beta(1-4)GlcNAc.

### Description of the Related Art

The carbohydrate structure attached to a peptide chain is known as a "glycan." The specific glycan structure present on a protein affects the solubility, intra- and inter-polypeptide association (e.g., tendency for aggregation and ability to correctly fold), and therefore its functional or enzymatic activity. In addition, the glycan may provide resistance to the peptide from proteolytic attack and the control of proteolysis leading to the conversion of inactive forms of the peptide to active forms or active forms into inactive forms. Importantly, terminal sialic acid residues present on the glycan molecule affect the half life of the peptide in the mammalian circulatory system. Thus, glycan structures provide methods to alter important pharmacokinetic properties of recombinant protein therapeutics.

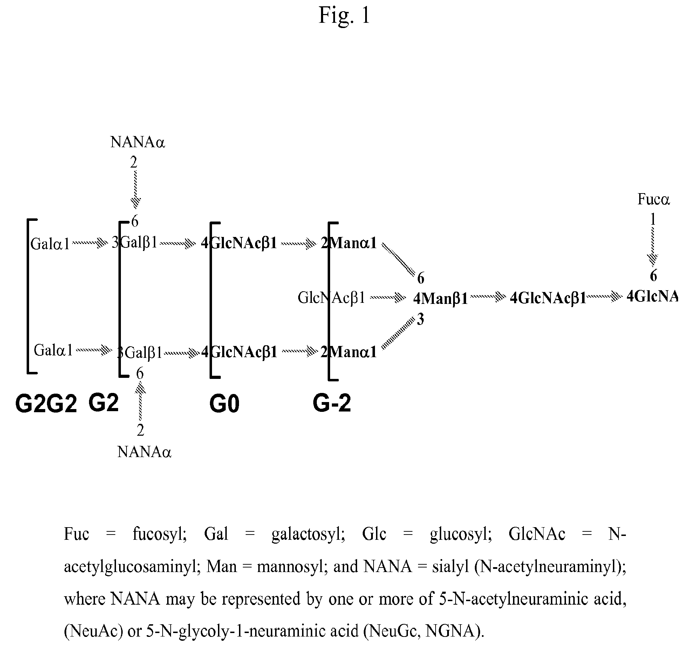

Antibodies are produced naturally and recombinantly as biopharmaceuticals in soluble glycoprotein form. All naturally produced antibodies possess glycans attached at conserved positions in the heavy chain constant regions, which position and structure vary with antibody isotype. Each isotype possesses a distinct array of N-linked oligosaccharide structures, which variably affect protein assembly, secretion or functional activity (Wright, A., and Morrison, S. L., Trends Biotech. 15:26-32 (1997)). In the mature IgG isotype antibody, the two complex biantennary oligosaccharides attached to an asparagine residue of the heavy chain are buried between the CH2 domains, forming extensive contacts with the polypeptide backbone. It has been found that their presence is essential for the antibody to mediate effector functions, such as ADCC (Lifely, M. R., et al., Glycobiology 5:813-822 (1995); Jefferis, R., et al., Immunol Rev. 163:59-76 (1998); Wright, A. and Morrison, S. L., *supra*)*.* The major structures found in human IgG and other recombinantly-produced IgGs are the complex biantennary structures with or without exposed Gal residues (Fig. 1). The biological significance of terminal Gal containing structures on the antibody functions has been studied in detail. The extent of galactosylation of antibodies is affected by age, gender, and disease (Raju, T.S., et al. Glycobiology 2000. 10(5): 477-86). In general, oligosaccharide structures are somewhat species-specific and vary widely.

WO 2009/146362 describes a yeast strain for the production of proteins with terminal alpha-1,3-linked galactose.

US 5,922,577 describes enzymatic synthesis of glycosidic linkages.

US 2004/0191256 describes methods and compositions for galactosylated glycoproteins.

Typically, there is heterogeneous processing of the core oligosaccharide structures attached at a particular glycosylation site such that even monoclonal antibody oligosaccharides exist as multiple glycoforms. Likewise, it has been shown that major differences in antibody glycosylation occur between antibody-producing cell lines, and even minor differences are seen for a given cell line grown under different culture conditions.

Antibodies expressed in some rodent cell lines (such as rodent myeloma derived host cells NS/0 and SP2/0) often contain oligosaccharides terminated with alpha-galactose residues. The galactose residues are linked to the penultimate galactose residues at a hydroxyl of the third sugar carbon position, alpha(1-3) linkage. Neither human nor hamster cells express the active alpha-galactosyltransferase and humans have up to 1% of circulating antibodies directed against the enzymatic product of alpha 1,3-galactosyltransferase (Gal alpha 1-3Gal beta 1-4GlcNAc), also called Galili antigen (Galili, U., Clark, M. R., Shohet, S. B., Buehler, J., and Macher, B. A. (1987) Proc. Natl. Acad. Sci. U. S. A. 84, 1369-1373). The absence of alpha 1-3Gal epitopes from human cells due to silencing of the gene for the enzyme alpha 1,3 galactosyltransferase, which participates in the glycosylation of cell membrane glycoconjugates in nonprimate mammals, prosimians, and New World monkeys, appears to have occurred in Old World primates 20-30 million years ago (Galili et al. 1988 J Biol Chem. 263(33):17755-62). The source of rejection of porcine organs transplanted to humans has also been traced to the alpha-Gal antigen.

Apart from the antigenic nature of the Gal alpha(1-3)Gal beta(1-4)GlcN Ac trisaccharide, the biological effect of alpha-galactosylated oligosaccharides on antibody function is unknown. Since oligosaccharides present in antibodies are highly heterogeneous, it is difficult to establish whether alpha-galactose present in therapeutic antibody preparations impacts the bioactivity. In one report, non-Fc-linked N-glycans present in the variable (antigen binding) region of a therapeutic antibody provided immunogenic (Chung et al. 2008 New Engl J Med 358:1109-17) and the reactive antigen was identified as Gal-alpha-1,3-Gal.

Therefore, a preparation of homogeneously alpha 1-3galactosylated antibodies that can be used to study the biological significance of alpha-galactose epitopes on antibody functions and pK would be of use in determining the biological impact of these glycans in therapeutic antibody preparations produced by non-primate host cells.

### SUMMARY OF THE INVENTION

The invention provides a method of obtaining a homogenously alpha-galactosylated antibody preparation by producing an alpha-galactosylated oligosaccharide structure comprising a terminal Gal-alpha(1,3)-Gal-beta(1-4)GlcNAc on an antibody, wherein the method comprises:
(a) admixing an antibody, an activated galactose, a divalent metal selected from the group consisting of Mn²⁺, Ca²⁺, and Zn²⁺, an alpha(1-3)galactosyltransferase, and a beta(1-4)galactosyltransferase in an aqueous medium within a single vessel to form an aqueous reaction medium; and
(b) maintaining said aqueous reaction medium at a pH of about 5 to about 10 at a temperature of about 25°C to about 40°C for a time period sufficient for said antibody to be glycosylated.

Herein disclosed is a method for synthesis of Gal alpha(1-3)Gal beta(1-4)GlcN Ac containing oligosaccharides in a single reaction. Herein also disclosed are substantially homogeneous preparations comprising Gal alpha(1-3)Gal beta(1-4)GlcN Ac containing oligosaccharide.

In one aspect for forming an alpha-galactosylated oligosaccharide structure comprising a terminal Gal-alpha(1,3)-Gal-beta(1-4)GlcNac includes the steps of
(a) admixing the following ingredients in an aqueous medium within a single vessel to form an aqueous reaction medium:
   i) a GlcNAc acceptor molecule;
   ii) a source of UDP-Gal;
   iii) a divalent metal selected from the group consisting of Mn²⁺, Ca²⁺, and Zn²⁺;
   iv) an alpha(1-3)galactosyltransferase; and
   v) a beta(1-4)galactosyltranferase; and
(b) maintaining said aqueous reaction medium at a pH value of about 5 to about 10 at a temperature of about 25°C to about 40°C for a time period sufficient for said acceptor to be glycosylated.

In one aspect the alpha-galactosylated oligosaccharide is a biantennary structure. In a specific aspect the alpha-galactosylated oligosaccharide biantennary structure is an N-glycan of a polypeptide. In an aspect the polypeptide is the heavy chain of an immunogloblulin.

The homogeneous preparations comprising Gal-alpha(1-3)Gal-beta(1-4)GlcNAc containing oligosaccharide may be used to study the antigenic nature of the terminal trisaccharide epitope and other biological responses to the presence of the epitope in various human and non-human systems. The preparations may be admixed to form a minor but defined component of the oligosaccharide preparation for such studies. The preparations may be used as starting material for preparations of oligosaccharides with greater complexity.

### BRIEF DESCRIPTION OF THE SEVERAL VIEWS OF THE DRAWING

**Fig. 1** shows the basic biantennary structure of major oligosaccharide structures found in either a naturally occurring and recombinant isolated IgG preparation, where the saccharide residues shown in bold face are core residues and those shown in normal font represent positions which vary based on the synthetic environment, such as the host cell origin, host cell nutritional environment, and post secretory processing or degradation: bisecting GlcNAc, alpha1-6 fucosylation of the core GlnNAc, and sialylation of galactosylated structures (alpha 2,6-sialylation).

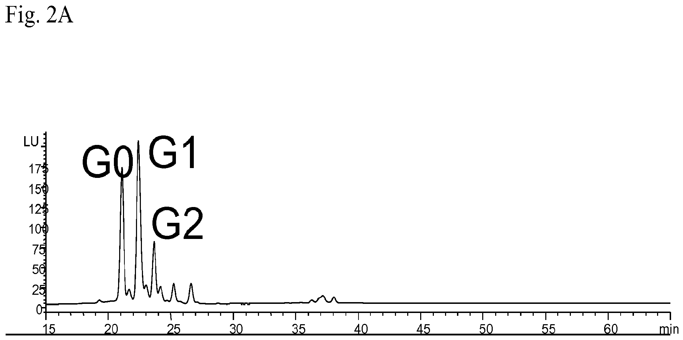

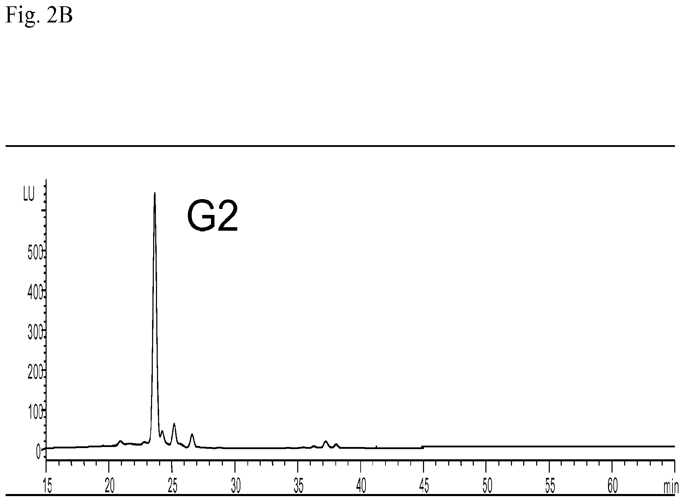

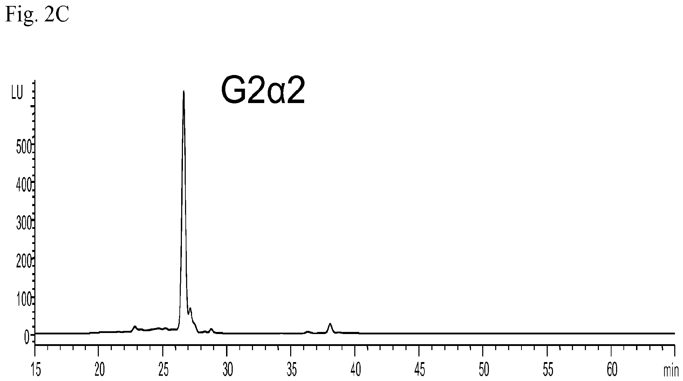

**Figs. 2A-2C** show chromatograms from a normal phase HPLC separation of oligosaccharides released from the starting preparation of IgG (Fig. 2A); the IgG after reaction with UDP-Gal in the presence of beta1,4galactosyltransferase (Fig. 2B); or the IgG after reaction with UDP-Gal in the presence of beta1,4galactosyltransferase and alpha-galactosyltransferase (Fig. 2C).

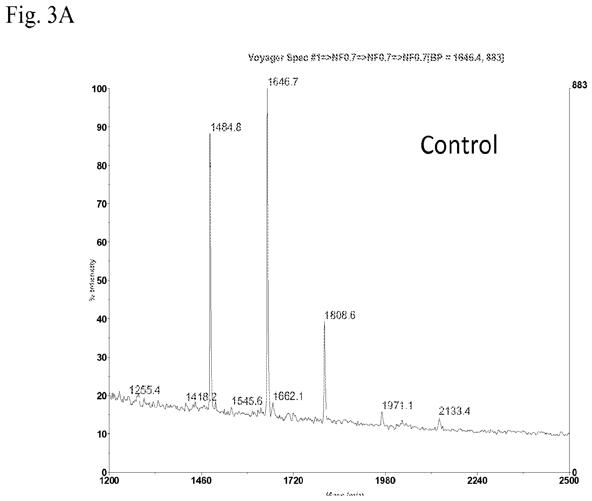

**Figs. 3A-3C** show a tracing from MALDI-TOF-MS analysis of oligosaccharides released from IgG samples.

### DETAILED DESCRIPTION OF THE INVENTION

### Abbreviations

α1,3GT, α-1,3-galactosyltransferase; α2,3ST, α-2,3-sialyltransferase; β1,4GT, β-1,4-galactosyltransferase; ADCC, antibody-dependent cellular cytotoxicity; CDC, complement-directed cytotoxicity; CMP-Sia, cytidine monophosphate, N-acetylneuraminic acid; fuc = fucosyl; gal = galactose; GalNac = N-acetylgalactose; Glc = glucosyl; IgG, immunoglobulin G; Man = mannosyl; MALDI-TOF-MS, matrix-assisted laser/desorption ionization time-of-flight mass spectrometry; MHX, mycophenolic acid, hypoxanthine, xanthine.; NANA, *N*-acetylneuraminic acid isomer of sialic acid; NGNA, *N*-glycolylneuraminic acid isomer of sialic acid; PNGase F, peptide *N*glycosidase F; RP-HPLC, reversed phase high-performance liquid chromatography; Sia, sialic acid; UDP-Gal, uridine diphosphate galactose; UDP-GlcNAc, uridine diphosphate *N-*acetylglucosamine.

### Definitions

The terms "antibody," "immunoglobulin," or "IgG" is intended to encompass antibodies, digestion fragments, specified portions and variants thereof, including, without limitation, antibody mimetics or comprising portions of antibodies that mimic the structure and/or function of an antibody or specified fragment or portion thereof, and retain Fc-mediated functions, including but not limited to: ligand binding, binding to Fc-receptors (e.g. FcγRI (CD64) FcγRIIA (CD32A), FcγRIIIA (CD16A) and FcRn), binding complement (e.g. C1q), ADCC and CDC.

The term "Fc-containing protein" or "Fc-containing molecule" as used herein refers to a monomeric, dimeric or heterodimeric protein having at least an immunoglobulin CH2 and CH3 domain, and preferably a dimerization domain, such as an immunoglobuline hinge region. The CH2 and CH3 domains can form at least a part of the dimeric region of the protein/molecule (e.g., antibody), wherein an N-linked glycosylatation site is present on one of the CH2 domains.

"Glycosylation sites" refer to amino acid residues which are recognized by a eukaryotic cell as locations for the attachment of sugar residues. The amino acids where carbohydrate, such as oligosaccharide, is attached are typically asparagine (N-linkage), serine (O-linkage), and threonine (O-linkage) residues. The specific site of attachment is typically signaled by a sequence of amino acids, referred to herein as a "glycosylation site sequence." The glycosylation site sequence for N-linked glycosylation is known as -Asn-X-Ser- or -Asn-X-Thr- (NXT), where X may be any of the conventional amino acids, other than proline. The predominant glycosylation site sequence for O-linked glycosylation is: -(Thr or Ser)-X-X-Pro-, where X is any conventional amino acid. The recognition sequence for glycosaminoglycans (a specific type of sulfated sugar) is -Ser-Gly-X-Gly, where X is any conventional amino acid. The terms "N-linked" and "O-linked" refer to the chemical group that serves as the attachment site between the sugar molecule and the amino acid residue. N-linked sugars are attached through an amino group; O-linked sugars are attached through a hydroxyl group. However, not all glycosylation site sequences in a protein are necessarily glycosylated; some proteins are secreted in both glycosylated and nonglycosylated forms, while others are fully glysosylated at one glycosylation site sequence but contain another glycosylation site sequence that is not glycosylated. Therefore, not all glycosylation site sequences that are present in a polypeptide are necessarily glycosylation sites where sugar residues are actually attached. The initial N-glycosylation during biosynthesis inserts the "core carbohydrate" or "core oligosaccharide" (Proteins, Structures and Molecular Principles, (1984) Creighton (ed.), W.H. Freeman and Company, New York).

The term "monoclonal antibody" as used herein is a specific form of Fc-containing fusion protein in which the ligand binding domain retains substantial homology to at least one of a heavy or light chain antibody variable domain of at least one species of animal antibody and the antibody is produced by single host cell type which may be a hybridoma or transfectoma but more typically, where the nucleic acids encoding the antibody have been recloned using standard recombinant methods and reintroduced into the host cell.

By "NANA" or "sialic acid" is meant a member of a family of nine-carbon carboxylated sugars. The most common member of the sialic acid family is N-acetyl neuraminic acid (2-keto-5-acetamido-3,5-dideoxy-D-glycero-D- galactononulopyranos-1-onic I acid (Neu5Ac, NeuAc, or NANA). A second member of the family is N-glycolylneuraminic acid (NGNA, Neu5Gc or NeuGc), in which the N-acetyl group of NeuAc is hydroxylated. This form is prevalent in glycoproteins from rodent and microbial sources. A third sialic acid family member is 2-keto-3-deoxy-nonulosonic acid (KDN) (Nadano et al. (1986) J. Biol. Chem. 261: 11550-11557; Kanamori et al., J. Biol. Chem. 265: 21811-21819 (1990)). Also included are 9-substituted sialic acids such as a 9-O-C -C6 acyl Neu5Ac like 9-O-lactyl-Neu5Ac or 9- O-acetyl-Neu5Ac, 9-deoxy-9-fluoro-Neu5Ac and 9 azido-9-deoxy-Neu5Ac. For review of the static acid family, see, e.g., Varki, Glycobiology 2: 25-40 (1992); Sialic Acids: Chemistry, Metabolism and Function, R. Schauer, Ed. (Springer Verlag, New York (1992)).

### Subject Glycans

The description relates to compositions which are oligosaccharide, also called "glycan" structures. Oligosaccharides are considered to have a reducing end and a non-reducing end, whether or not the saccharide at the reducing end is in fact a reducing sugar. In accordance with accepted nomenclature, oligosaccharides are depicted herein with the non-reducing end on the left and the reducing end on the right. All oligosaccharides described herein are described with the name or abbreviation for the non-reducing saccharide (e.g., Gal), followed by the configuration of the glycosidic bond (α or β), the ring bond, the ring position of the reducing saccharide involved in the bond, and then the name or abbreviation of the reducing saccharide (e.g., GlcNAc). The linkage between two sugars may be expressed, for example, as 1,3, 1→3, or (1-3). Each saccharide is a pyranose.

The oligosaccharide structures disclosed in the present description occur on an antibody expressed as N-linked oligosaccharides. "N-linked glycosylation" refers to the attachment of the carbohydrate moiety via GlcNAc to an asparagine residue in a polypeptide or lipid chain. The N-linked oligosaccharides on mammalian antibodies contain a common Man alpha(1-6)[Man alpha(1- 3)]Manbeta(1-4)GlcNAcbeta(1-4)GIcNAcbeta-R "core structure" also referred to as G-2 (Fig. 1). Therefore, in the core structure described, R represents an asparagine residue of the produced glycoprotein linked to the first saccharide of the carbohydrate: 2-acetamido-N-(L-aspart-4-yl)-2-deoxy-b-D-glucopyranosylamine, i.e. *N*⁴-(N-acetyl-b-D-glucosaminyl)asparagine, which is also abbreviated to (GlcNAc-)Asn (parentheses here around the carbohydrates placed next to the symbol for an the asparagine residue indicates substitution is on the N at the fourth atom which is the side chain amine). Oligosaccharides having branched chains are considered complex carbohydrates and the present description relates to complex biantennary carbohydrate structures also referred to as the glycan portion of a glycoprotein, such as those attached to the CH2 domain of immunoglobulins.

The natural modification of polypeptides with oligosaccharides occurs in the golgi apparatus of eukaryotic cells, particularly eukaryotic cells capable of adding an N-linked "core oligosaccharide" containing at least one mannose residue and/or capable of adding an O-linked sugar, to at least one glycosylation site sequence in at least one polypeptide expressed in said cell, particularly, a secreted protein. Thus, cells capable of forming glycoproteins contain at least one glycosyltransferase that catalyzes the attachment of a sugar residue to a glycosylating site sequence in a protein or polypeptide. Mammalian cells are typically capable of glycosylating proteins while other eukaryotic cells, such as insect cells and yeast, may glycosylate secreted proteins but with alternative or truncated structures as compared to those produced by mammalian cells.

### Compositions

The product of the method of the disclosure is a substantially homogeneous preparation comprising an alpha-galactosylated oligosaccharide structure comprising a terminal Gal-alpha(1,3)-Gal-beta(1-4)GlcNAc. The alpha-galactosylated oligosaccharide structure may be linked to proteins or lipids, through amine or hydroxyl functionalities present on proteins on the side chain of asparagine, serine, or threonine residues and hydroxyl groups of terpenoids or ceramide, sphingoid, such as prenyl phosphate.

The description also relates to complex biantennary structures comprising α1,3-linkage Gal, optionally, with α2,6-linked NANA. In one embodiment, the structure produced by the method of the invention is shown in the formula below (I):

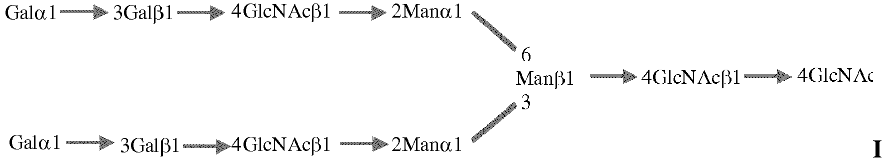

In a different aspect the structure produced by the method of the invention is shown in the formula below (II):

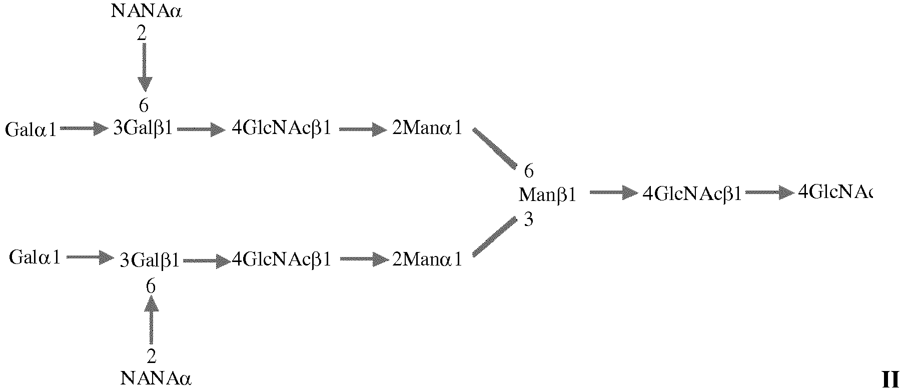

It will be obvious to those skilled in the art that included within the present invention are variations of formula I and II that are possible along with variants, such as those depicted in Fig. 1, which include the presence of core fucose and bisecting GlcNAc.

### Methods of Making the Compositions

A number of glycosyltransferases have been described and, in some cases, methods whereby the enzymes may be used concurrently instead of sequentially to affect the synthesis of a bisaccharide of stereo- and region-specificity. Over 200 glycsosyltransferases from various sources have been identified and the ability to select compatible combinations for the directed synthesis of specific oligosaccharide structures has not been exhaustively explored. The invention describes that by selection of galactosyltransferase enzymes with predetermined specificity, it is possible to transfer two molecules of galactose in series in a single reaction to a substrate comprising a terminal GlcNac forming the specific trisaccharide structure Gal α(1-3)Galp(1-4)GlcNAc.

In one aspect for forming an alpha-galactosylated oligosaccharide structure comprising a terminal Gal-alpha(1,3)-Galbeta(1-4)GlcNAc includes the steps of:
(a) admixing the following ingredients in an aqueous medium within a single vessel to form an aqueous reaction medium:
   vi) an GlcNAc acceptor molecule;
   vii) a source of UDP-Gal;
   viii) a divalent metal selected from the group consisting of Mn²⁺, Ca²⁺, and Zn²⁺;
   ix) an alpha(1-3)galactosyltransferase; and
   x) a beta(1-4)galactosyltranferase; and
(b) maintaining said aqueous reaction medium at a pH of about 5 to about 10 at a temperature of about 25°C to about 40°C for a time period sufficient for said acceptor to be glycosylated.

In one aspect the galactosyltransferase is isolated from a natural source. For example, bovine milk beta-1,4 galactosyltransferase is a common source of commercially available enzyme. Recombinant forms of bovine, porcine, and other galactosyltransferases are also available. Recombinant alpha-1,3 galactosyltransferases have been previously expressed as complete proteins or as the soluble extracellular domain which is a fully active soluble enzyme (Henion, T. R., Macher, B. A., Anaraki, F., and Galili, U. (1994) Glycobiology 4, 193-201).

The divalent metal specificity for activating the alpha(1-3) and beta(1-4)-galactosyltransferases is similar or at least overlapping in vitro environments and includes Mn²⁺, Zn²⁺, and Co²⁺ (Zhang et al. 2001 J. Biol. Chem., 276(15): 11567-11574). The metal or metals are present at 1-25 mM.

Exemplary galactosyltransferases and glycosaminoglycan galactosyltransferase of *Dictyostelium discoideum* (EC 2.4.1.74), mammalian glucosaminylgalactosylglucosylceramide β-galactosyltransferase (EC 2.4.1.86); β-*N-*acetylglucosaminyl-glycopeptide β-1,4-galactosyltransferase (E.C. No. 2.4.1.38) also called N-acetyllactosamine synthase (EC 2.4.1.22) capable of catalyzing the reaction UDP-galactose + *N*-acetyl-β-D-glucosaminylglycopeptide = UDP + β-D-galactosyl-(1→4)-*N*-acetyl-β-D-glucosaminylglycopeptide In another aspect the galactosyltrasferease is also called N-acetyllactosamine synthase (EC 2.4.1.22) and is capable of catalyzing the transfer of galactose from UDP-galactose to N-acetylglucosamine.

The α(1,3) galactosyltransferase (E.C. No. 2.4.1.151) especially that of calf thymus (Blanken et al. J Biol Chem. 1985 Oct 25;260(24): 12927-34) or porcine + β-D-galactosyl-*N*-acetylglucosamine- α(1,3)D-galactosyltransferase is capable of catalyzing the formation of the trisaccharide antigen, Gal α(1-3)Galβ(1-4)GlcNAc. The α(1,3)D-galactosyltransferases useful in the method of the invention are capable of catalyzing the reaction:
UDP-galactose + β-D-galactosyl-(1→4)-*N*-acetyl-β-D-glucosaminylglycopeptide = UDP + α(1→3)galactosyl-β-D-galactosyl-(1→4)-*N*-acetyl-β-D-glucosaminylglycopeptide

For production of the structure of formula II, a NANA transferring enzyme can be used, such enzymes include Gal-β-1,4- GlcNAc α-2,6 sialyltransferase (See, Kurosawa et al. Eur. J. Biochem. 219: 375-381 (1994)) and US Pat. 7,220,555).

Other glucosyltransferases particularly useful in preparing oligosaccharides acceptor molecules of invention are the mannosyltransferases including α(1,2) mannosyltransferase, α(1,3) mannosyltransferase, β(1,4) mannosyltransferase, Dol-P-Man synthase, OCh1, and Pmt1.

Still other glucosyltransferases include N-acetylgalactosaminyltransferases including α(1,3) N-acetylgalactosaminyltransferase, β(1,4) N-acetylgalactosaminyltransferases (Nagata et al. J. Biol. Chem. 267:12082- 12089 (1992) and Smith et al. J. Biol Chem. 269:15162 (1994)) and polypeptide N-acetylgalactosaminyltransferase (Homa et al. J. Biol Chem. 268:12609 (1993)). Suitable N-acetylglucosaminyltransferases include GnTI (2.4.1.101, Hull et al., BBRC 176:608 (1991)), GnTII, and GnTIII (Ihara et al. J. Biolchem. 113:692 (1993)), GnTV (Shoreiban et al. J. Biol. Chem. 268: 15381 (1993)).

For those aspects in which the method is to be practiced on a commercial scale, it can be advantageous to immobilize the glycosyltransferase on a support. This immobilization facilitates the removal of the enzyme from the batch of product and subsequent reuse of the enzyme. Immobilization of glycosyltransferases can be accomplished, for example, by removing from the transferase its membrane-binding domain, and attaching in its place a cellulose-binding domain. One of skill in the art will understand that other methods of immobilization can also be used and are described in the available literature.

The glycosyltransferase used is specific for both the transferred glycosyl group and the acceptor to which the glycosyl group (Gal or GlcNAc) is transferred. When synthesizing oligosaccharides from scratch, the acceptor substrates can essentially be any monosaccharide or oligosaccharide having a terminal saccharide residue for which the particular glycosyltransferase exhibits specificity, and the substrate may be substituted at the position of its non-reducing end. Thus, the glycoside acceptor may be a monosaccharide, an oligosaccharide, a fluorescent-labeled saccharide, or a saccharide derivative, such as an aminoglycoside antibiotic, a ganglioside, a glycolipid, or a glycoprotein including antibodies and other Fc-containing proteins. In one group of preferred embodiments, the glycoside acceptor is an oligosaccharide, which when beta-galactosylated will comprise the disaccharide unit Galβ(1-4)GlcNAc, thereby acting as an acceptor for the alpha-galactosyltransfersas. The saccharide or oligosaccharide acceptor is preferably,
GlcNAc,
GlcNAcβ(1-2)Man,
GlcNAcβ(1-2)Manα(1-3)Man,
GlcNAcβ(1-2)Manα(1,6)Man,
GlcNAcβ(1-2)Manα(1,6)Man β(1-4)GlcNAc,
GlcNAcβ(1-2)Manα(1,6)Man β(1-4)GlcNAc, β(1-4)GlcNac
GlcNAcβ(1-2)Manα(1,6)Man β(1-4)GlcNAc, β(1-4)GlcNac-R, or
GlcNAcβ(1-2)Manα(1,6)[ Galβ(1-4)GlcNAcβ(1-2)Manα(1-3)]Man β(1-4)GlcNAc, β(1-4)GlcNac-R.

In a particular aspect the oligosaccharide acceptor, is linked to R, where R is an asparagines residue within the CH2 domain of an Fc-containing protein. In another embodiment, the non-reducing terminal sugar may be substititute with a reporter group or be attached to a lipid such as an aminophospholipid.

The glycosyltransferase will also have specificity for the donor sugar nucleotide. In the case of the galactosyltransferases, the donor sugar nucleotide may be UDP-Gal. The use of activated sugar substrate, i.e., sugar-nucleoside phosphate, can be circumvented by using a regenerating reaction concurrently with the glycotransferase reaction (also known as a recycling system). For example, as taught in, e.g., U.S. Pat. 5,516,665; a uridine diphosphate recycling system that includes (a) UDP, UTP or both, (b) a phosphate donor, and (c) a kinase to transfer a phosphate group from the phosphate donor to UDP to form UTP, wherein each of the enzymes is present in a catalytic amount. Either or both of UDP and UTP can be present inasmuch as UDP is converted into UTP, and after the glycosyl transfer reaction, UDP is formed again. Because UDP and UTP interconvert and are reused, the total amount of one or the other is usually discussed rather than amounts for both. The phosphate donor of the regenerating system is a phosphorylated compound, the phosphate group of which can be used to phosphorylate UDP to form UTP. The only limitation on the selection of a phosphate donor is that neither the phosphorylated nor the dephosphorylated forms of the phosphate donor substantially interferes with any of the reactions involved in the formation of the glycosylated acceptor saccharide. Phosphate donors are phosphoenolpyruvate (PEP) and acetyl phosphate (AcOP).

Yet another system for forming UDP-gal is taught in US 5,728,554 and includes a donor substrate recycling system comprising at least 1 mole of glucose-1-phosphate per each mole of substrate oligosaccharide, a phosphate donor, a kinase capable of transferring phosphate from the phosphate donor to nucleoside diphosphates, and a pyrophosphorylase capable of forming UDP-glucose from UTP and glucose-1-phosphate and catalytic amounts of UDP and a UDP-galactose-4-epimerase. This system can be used with α(1,3) galactosyltransferase (E.C. No. 2.4.1.151) and β(1,4) galactosyltransferase (E.C. No. 2.4.1.38).

An alternative method of preparing oligosaccharides is through the use of a glycosyltransferase and activated glycosyl derivatives as donor sugars obviating the need for sugar nucleotides as donor sugars as taught in U.S. Pat. 5,952,203. The activated glycosyl derivatives act as alternates to the naturally-occurring substrates, which are expensive sugar-nucleotides, usually nucleotide diphosphosugars or nucleotide monophosphosugars in which the nucleotide phosphate is α-linked to the 1-position of the sugar.

Activated glycoside derivatives which are useful include an activated leaving group, such as, for example, fluoro, chloro, bromo, tosylate ester, mesylate ester, triflate ester and the like. Preferred embodiments of activated glycoside derivatives include glycosyl fluorides and glycosyl mesylates, with glycosyl fluorides being particularly preferred. Among the glycosyl fluorides, α-galactosyl fluoride, α-mannosyl fluoride, α-glucosyl fluoride, α-fucosyl fluoride, α-xylosyl fluoride, α-sialyl fluoride, alpha-N-acetylglucosaminyl fluoride, α-N-acetylgalactosaminyl fluoride, β-galactosyl fluoride, β-mannosyl fluoride, β-glucosyl fluoride, β-fucosyl fluoride, β-xylosyl fluoride, beta-sialyl fluoride, β-N-acetylglucosaminyl fluoride and β-N-acetylgalactosaminyl fluoride are most preferred.

Glycosyl fluorides can be prepared from the free sugar by first acetylating the sugar and then treating it with HF/pyridine. Acetylated glycosyl fluorides may be deprotected by reaction with mild (catalytic) base in methanol (e.g., NaOMe/MeOH). In addition, many glycosyl fluorides are commercially available. Other activated glycosyl derivatives can be prepared using conventional methods known to those of skill in the art. For example, glycosyl mesylates can be prepared by treatment of the fully benzylated hemiacetal form of the sugar with mesyl chloride, followed by catalytic hydrogenation to remove the benzyl groups.

Suitable analogs include, for example, nucleoside sulfates and sulfonates. Still other analogs include simple phosphates, for example, pyrophosphate.

One procedure for modifying recombinant proteins produced, in e.g., murine cells wherein the hydroxylated form of sialic acid predominates (NGNA), is to treat the protein with sialidase, to remove NGNA-type sialic acid, followed by enzymatic galactosylation using the reagent UDP-Gal and beta1,4 Galtransferase to produce highly homogeneous G2 glycoforms.

An alternative approach for preparing sublots of an Fc-containing protein that differ in α-galactose content of the oligosaccharides in the Fc region is to treat a portion of an Fc-containing protein preparation with sialidase enzyme, thereby removing sialic acids.

### Methods of Using the Invention

The method the invention can be used to modify polypeptides having an consensus glycosylation sequence (NXT) having a core glycan structure known as GO (Fig. 1) to structures containing beta Gal residues (G2) further comprising at least one alpha1-3 galactosylated saccharides (G2G1 or G2G2) as shown in Fig. 1 and below (I).

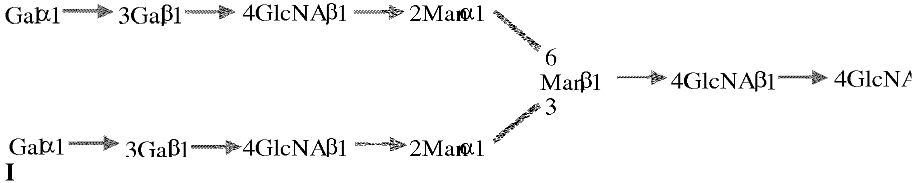

The disclosure further relates to preparations of IgG which comprise glycan structures which are substantially homogeneously in the form of G2G2 as shown in (I) which may further be fucosylated at the core GlcNac, or may have bisecting beta-1-4 N-acetyl aminoglucosylated at the core mannose of the structure, or may be sialylated at the same galactose residue which is alpha-galactosylated, by an alpha 2-6 linkage but not alpha-2-3 sialylated at the same galactose residue which is alpha-galactosylated.

The compositions prepared by the process of the invention, are useful as therapeutic compositions wherein a substantially homogeneous preparation of IgG molecules is desired having glycans in the G2G2 configuration. The method of the invention may be used to modify glycoproteins that interact with receptors. In particular, the invention relates to the modification of the glycan groups on a therapeutic antibody capable of interaction with Fc-receptors and producing modified therapeutic proteins, e.g., antibodies, such that the composition of the oligosaccharide chains may be optimized for one or more biological activities in vivo.

The compositions prepared by the process of the invention may be subjected to further biologic or chemical processing or modification. For example, antibodies prepared with glycan structures in the G2G2 configuration can be modified to include alpha-2,6-sialylation. Higher order structures or modifications, such as PEGylation or lipidation, of one or more of the saccharide residues of compositions produced by the method of the enzymatic method of the invention are encompassed by the disclosure.

### EXAMPLE 1: GALACTOSYLATION OF ANTIBODY SAMPLES

A method of preparation of IgG substantially in the G2 glycoform is described.

Bovine β-1,4-galactosyltransferase and UDP-Gal were obtained from Sigma Chemical Co. (St. Louis, MO). PNGase F was obtained from New England Biolabs (Beverly, MA) or from Prozyme (San Leandro, CA) or from Selectin BioSciences (Pleasant Hill, CA). NAP-5 and HiTrap protein A columns were obtained from Pharmacia Biotech (Piscataway, NJ). All other reagents were of analytical grade. Recombinant IgGs comprising a human Fc-domain were produced at Centocor Research & Development, Inc. (Radnor, PA).

The IgG samples in 100 mM MES buffer (pH 7.0) (approximately 10 mg in 1.0 mL of buffer) were treated with 50 milliunits of bovine β1,4-galactosyltransferase (from Sigma), 5 µmol of UDP-Gal, and 5 µmol of MnCl₂ at 37°C for 24 hours. Another aliquot of enzyme and UDP-Gal was added and the mixture was incubated for an additional 24 hours at 37°C. The alpha-galactosylated IgG samples were purified using a HiTrap protein A column. The oligosaccharides were released by treating IgGs with PNGase F and characterized the released oligosaccharides by MALDI-TOF-MS and by NP-HPLC (normal phase HPLC).

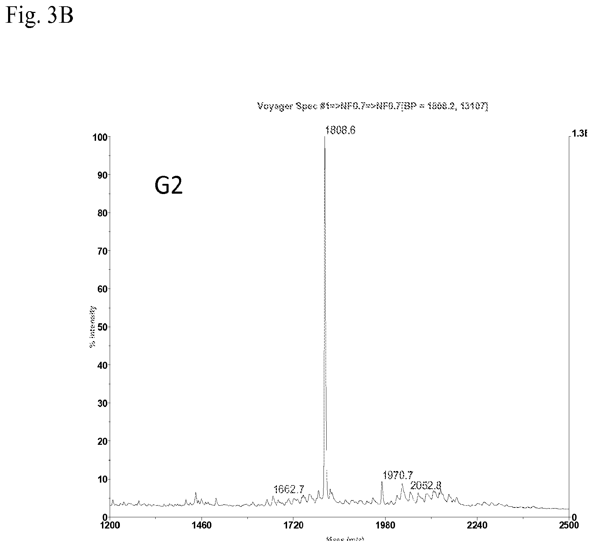

The MALDI-TOF-MS analysis of glycans released from starting IgG sample (control) showed the presence of 45% GO, 50% G1 and 5% G2 glycans along with minor amounts of other glycans (Fig. 3A). The NP-HPLC analysis of glycans released from untreated IgG sample showed no appreciable amounts of sialylated glycans and confirmed the presence of GO, G1 and G2 as major glycans (Fig. 2A). Thus, after incubation of the samples with Bovine β-1,4-galactosyltransferase and UDP-Gal (obtained from Sigma), both MALDI-TOF-MS and NP-HPLC analyses of glycans released from the galactosylated IgG sample showed the presence of only G2 glycan (Figs. 2B and 3B) and the absence of G0 and G1 glycans suggesting that the galactosylation was complete.

### EXAMPLE 2: α-GALACTOSYLATION OF ANTIBODY SAMPLES

A homogeneous preparation containing Gal(alpha1-3)Galβ1,4 linkages in an IgG preparation comprising the glycan of formula I was prepared in a single reaction step using non-primate enzymes

The reagents were as described in Example 1 with the addition of recombinant porcine α-galactosyltransferase was obtained from Calbiochem (San Diego, CA)

IgG samples in 100 mM MES buffer (pH 7.0) (∼10 mg in 1.0 mL of buffer) were treated with 50 milliunits of each of bovine β1,4-galactosyltransferase (from Sigma) and recombinant rat liver α1,3-galactosyltransferase (from CalBiochem) in the presence of 5 µmol of UDP-Gal and 5 µmol of MnCl₂ at 37 °C. After 24 hr of incubation, another aliquot of bovine β1,4-galactosyltransferase and recombinant rat liver α1,3-galactosyltransferas along with 5 µmol of UDP-Gal were added. The mixture was incubated for an additional 24 hr at 37 °C. The beta-galactosylated and α-galactosylated IgG samples were purified using a HiTrap protein A column. The oligosaccharides were released from IgGs by treating with PNGase F and characterized the released oligosaccharides by MALDI-TOF-MS and by NP-HPLC.

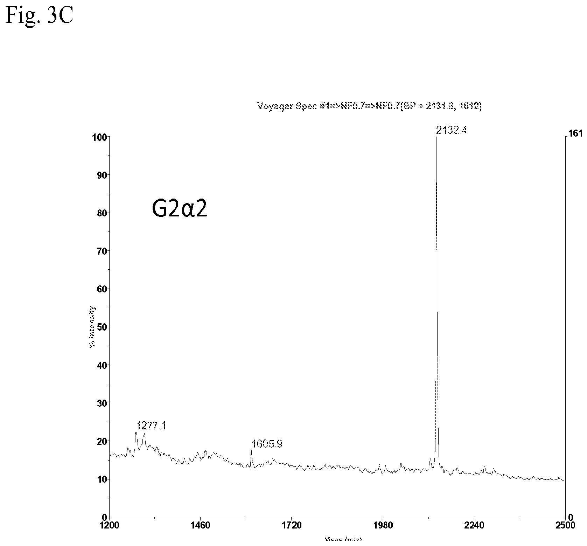

Both MALDI-TOF-MS and NP-HPLC analyses of glycans released from the treated IgG sample showed the presence of only a-galactosylated structure (Figs. 2C and 3C) i.e., G2α2 (Formula 1) and the absence of GO, G1 and G2 structures suggesting that the α-galactosylation of IgG is complete.

## Claims

1. A method of obtaining a homogenously alpha-galactosylated antibody preparation by producing an alpha-galactosylated oligosaccharide structure comprising a terminal Gal-alpha(1,3)-Gal-beta(1-4)GlcNAc on an antibody, wherein the method comprises:
(a) admixing an antibody, an activated galactose, a divalent metal selected from the group consisting of Mn²⁺, Ca²⁺, and Zn²⁺, an alpha(1-3)galactosyltransferase, and a beta(1-4)galactosyltransferase in an aqueous medium within a single vessel to form an aqueous reaction medium; and
(b) maintaining said aqueous reaction medium at a pH of 5 to 10 at a temperature of 25°C to 40°C for a time period sufficient for said antibody to be glycosylated.

2. The method of claim 1 wherein the alpha(1-3)galactosyltransferase is porcine α-galactosyltransferase.

3. The method of claim 1 or 2 wherein the divalent metal salt is Mn²⁺.

4. The method of any of claims 1 to 3 wherein the activated galactose is uridine diphosphate-galactose (UDP-galactose).

5. The method of any of claims 1 to 4 wherein the beta(1-4)galactosyltransferase is a mammalian beta1-4, galactosyl transferase.

6. The method of any of claims 1 to 5 wherein the reaction temperature is 37°C, the divalent metal is Mn²⁺ at a concentration of 5 mM, the UDP- galactose concentration is 5mM and the beta(1-4)galactosyltransferase concentration is 50 mUnit/ml.

7. The method of any of claims 1 to 6 wherein the antibody is an IgG.

8. The method of any of claims 1 to 7, wherein the saccharide acceptor is selected from the group consisting of GlcNAc, GlcNAcβ(1-2)Man, GlcNAcβ(1-2)Manα(1-3)Man, GlcNAcβ(1-2)Manα(1,6)Man, GlcNAcβ(1-2)Manα(1,6)Man β(1-4)GlcNAc, GlcNAcβ(1-2)Manα(1,6)Man β(1-4)GlcNAc, β(1-4)GlcNac, GlcNAcβ(1-2)Manα(1,6)Man β(1-4)GlcNAc, β(1-4)GlcNac-R, and GlcNAcβ(1-2)Manα(1,6)[ Galβ(1-4)GlcNAcβ(1-2)Manα(1-3)]Man β(1-4)GlcNAc, β(1-4)GlcNac-R.

9. The method of any of claims 1 to 8 wherein the glycan comprises alpha-2,6-sialic acid residues.

## Patentansprüche

1. Verfahren zum Erhalten eines homogen alpha-galactosylierten Antikörperpräparats durch Herstellen einer alpha-galactosylierten Oligosaccharid-Struktur, die ein terminales Gal-alpha(1,3)-Gal-beta(1-4)GlcNAc an einem Antikörper umfasst, wobei das Verfahren Folgendes umfasst:
(a) Mischen eines Antikörper, einer aktivierten Galactose, eines zweiwertigen Metalls, das ausgewählt ist aus der Gruppe bestehend aus Mn²⁺, Ca²⁺ und Zn²⁺, einer alpha (1-3)Galactosyltransferase und einer beta(1-4)Galactosyltransferase in einem wässrigen Medium in einem einzigen Gefäß, um ein wässriges Reaktionsmedium zu bilden; und
(b) Halten des wässrigen Reaktionsmediums bei einem pH-Wert von 5 bis 10 bei einer Temperatur von 25 °C bis 40 °C über einen Zeitraum, der zur Glycosylierung des Antikörpers ausreichend ist.

2. Verfahren nach Anspruch 1, wobei es sich bei der alpha(1-3)Galactosyltransferase um eine α-Galactosyltransferase des Schweins handelt.

3. Verfahren nach Anspruch 1 oder 2, wobei es sich bei dem Salz eines zweiwertigen Metalls um Mn²⁺ handelt.

4. Verfahren nach einem der Ansprüche 1 bis 3, wobei es sich bei der aktivierten Galactose um Uridindiphosphat-Galactose (UDP-Galactose) handelt.

5. Verfahren nach einem der Ansprüche 1 bis 4, wobei es sich bei der beta(1-4)Galactosyltransferase um eine beta(1-4)Galactosyltransferase eines Säugers handelt.

6. Verfahren nach einem der Ansprüche 1 bis 5, wobei die Reaktionstemperatur bei 37 °C liegt, es sich bei dem zweiwertigen Metall um Mn²⁺ in einer Konzentration von 5 mM handelt, die UDP-Galactose-Konzentration bei 5 mM liegt und die beta(1-4)Galactosyltransferase-Konzentration bei 50 mUnits/ml liegt.

7. Verfahren nach einem der Ansprüche 1 bis 6, wobei es sich bei dem Antikörper um ein IgG handelt.

8. Verfahren nach einem der Ansprüche 1 bis 7, wobei der Saccharid-Akzeptor ausgewählt ist aus der Gruppe bestehend aus GlcNAc, GlcNAcß(1-2)Man, GlcNAcβ(1-2)Manα(1-3)Man, GlcNAcβ(1-2)Manα(1,6)Man, GlcNAcβ(1-2)Manα(1,6)Manβ(1-4)GlcNAc, GlcNAcβ(1-2)Manα(1,6)Manβ(1-4)GlcNAc, β(1-4)GlcNac, GlcNAcβ(1-2)Manα(1,6)Manβ(1-4)GlcNAcβ(1-4)GlcNac-R und GlcNAcβ(1-2)Manα(1,6)[Galβ(1-4)GlcNAcβ(1-2)Manα(1-3)]Manβ(1-4)GlcNAc, β(1-4)GlcNac-R.

9. Verfahren nach einem der Ansprüche 1 bis 8, wobei das Glycan alpha-2,6-Sialsäure-Reste umfasst.

## Revendications

1. Procédé d'obtention d'une préparation d'anticorps alpha-galactosylé de façon homogène par production d'une structure d'oligosaccharide alpha-galactosylé comprenant un Gal-alpha(1,3)-Gal-bêta(1-4)GlcNAc terminal sur un anticorps, le procédé comprenant :
(a) le mélange d'un anticorps, d'un galactose activé, d'un métal divalent choisi dans le groupe constitué de Mn²⁺, Ca²⁺ et Zn²⁺, d'une alpha (1-3) galactosyltransférase, et d'une bêta(1-4)galactosyltransférase dans un milieu aqueux dans un récipient unique pour former un milieu de réaction aqueux ; et
(b) le maintien dudit milieu de réaction aqueux à un pH de 5 à 10 à une température de 25 °C à 40 °C pendant une durée suffisante pour que ledit anticorps soit glycosylé.

2. Procédé selon la revendication 1, dans lequel l'alpha(1-3)galactosyltransférase est une α-galactosyltransférase porcine.

3. Procédé selon la revendication 1 ou 2, dans lequel le sel de métal divalent est Mn²⁺.

4. Procédé selon l'une quelconque des revendications 1 à 3, dans lequel le galactose activé est l'uridine diphosphate-galactose (UDP-galactose).

5. Procédé selon l'une quelconque des revendications 1 à 4, dans lequel la bêta(1-4)galactosyltransférase est une bêta(1-4)galactosyltransférase de mammifère.

6. Procédé selon l'une quelconque des revendications 1 à 5, dans lequel la température de réaction est 37 °C, le métal divalent est Mn²⁺ à une concentration de 5 mM, la concentration d'UDP-galactose est de 5 mM et la concentration de bêta(1-4)galactosyltransférase est de 50 mUnité/ml.

7. Procédé selon l'une quelconque des revendications 1 à 6, dans lequel l'anticorps est un IgG.

8. Procédé selon l'une quelconque des revendications 1 à 7, dans lequel l'accepteur de saccharide est choisi dans le groupe constitué de GlcNAc, GlcNAcβ(1-2)Man, GlcNAcβ(1-2)Manα(1-3)Man, GlcNAcβ(1-2)Manα(1,6)Man, GlcNAcβ(1-2)Manα(1,6)Manβ(1-4)GlcNAc, GlcNAcβ(1-2)Manα(1,6)Manβ(1-4)GlcNAc, β(1-4)GlcNac, GlcNAcβ(1-2)Manα(1,6)Manβ(1-4)GlcNAc, β(1-4)GlcNac-R, et GlcNAcβ(1-2)Manα(1,6)[Galβ(1-4)GlcNAcβ(1-2)Manα(1-3)]Manβ(1-4)GlcNAc, β(1-4)GlcNac-R.

9. Procédé selon l'une quelconque des revendications 1 à 8, dans lequel le glycane comprend des résidus acide alpha-2,6-sialique.
